# EUROPEAN PATENT SPECIFICATION

(11) **EP 3 275 412 B1**
(45) Date of publication and mention of the grant of the patent: **20.11.2019**
(21) Application number: 15896394.2
(22) Date of filing: 26.06.2015
(51) Int. Cl.: A61F 13/15, A61F 13/49

(54) **APPARATUS FOR MANUFACTURING ABSORBENT FOR ABSORBENT ARTICLE**
VORRICHTUNG ZUR HERSTELLUNG EINES ABSORBENS FÜR EINEN SAUGFÄHIGEN ARTIKEL
APPAREIL DE FABRICATION D'ABSORBANT POUR ARTICLE ABSORBANT

(43) Date of publication of application: 31.01.2018
(73) Proprietor: Unicharm Corporation, Ehime 799-0111 (JP)
(72) Inventor: TSUKUDA, Atsushi, Kanonji-shi Kagawa 769-1602 (JP); TADA, Hiroaki, Kanonji-shi Kagawa 769-1602 (JP); MIYOSHI, Hiroyuki, Kanonji-shi Kagawa 769-1602 (JP)
(74) Representative: Wallinger Ricker Schlotter Tostmann
(86) International application number: PCT/JP2015/068560
(87) International publication number: WO 2016/208076

(56) References cited:
- WO-A1-2007/020562
- JP-A- S63 274 448
- JP-A- 2010 035 701
- JP-A- 2013 123 513
- US-A1- 2012 056 357
- US-B2- 8 616 867

## Description

### Technical Field

The present invention pertains to an apparatus to manufacture an absorbent body of an absorbent article.

### Background Art

Conventionally, an apparatus to manufacture an absorbent body of an absorbent article is disclosed, which includes a freely rotatable suction drum (a drum device) which accumulates a material of the absorbent body supplied through a cover duct (a flow housing) on a molding portion (a web forming layer) disposed on a circumferential surface (for example, refer to Patent Document 1).

Further, conventionally, the outer shape (the planar shape) of an absorbent body of an absorbent article was generally a simple one of a substantially rectangular shape or an hourglass shape in which the end portions in the longitudinal direction were widened. However, today, in order to improve the functionality such as absorption performance, diffusion performance, and the like, of an absorbent article, it is required to manufacture an absorbent body having a complicated outer shape.

As mentioned above, when manufacturing an absorbent body having a complex planar shape, a pattern plate which has a shape that is complementary to an outer shape of the absorbent body and in which a pattern hole that penetrates through in a thickness direction is formed, and which is provided in the circumferential surface of a suction drum, is generally used. In this case, the molding portion is formed inside the pattern hole of the pattern plate.

### Citation List

### Patent Literature

PTL 1: Japanese Unexamined Patent Publication No. S62-206071

### Summary of Invention

### Technical Problem

However, when forming an absorbent body of an absorbent article having such a complicated outer shape, the shape of the pattern hole of the pattern plate which has the shape that is complementary to the outer shape of the absorbent body also becomes complicated. Accordingly, in some cases, the material of the absorbent body may be accumulated on a part of the pattern hole. Further, when a part of the edge portions of the pattern hole has a width direction component perpendicular to the rotation direction of the suction drum, there may be cases in which the supply of the material of the absorbent body is unbalanced in the vicinity of the edge portions, due to the edge portions generating an air flow toward the rotation direction. As a result, there may be cases in which an absorbent body having a desired outer shape and basis weight distribution cannot be formed.

It is therefore an object of the present invention to provide an apparatus capable of manufacturing an absorbent body having a desired outer shape and basis weight distribution, even in a case of manufacturing an absorbent body of an absorbent article having a complicated outer shape.

US 8,616,867 B2 discloses an apparatus for forming a batt of particulate material including a vacuum zone, and air impermeable and permeable fence zones adjacent to the vacuum zone. The air permeable fence zone may comprise an ambient air entry conduit in communication with an ambient air exit port.

### Solution to Problem

To solve the above described problem, the present invention provides an apparatus to manufacture an absorbent body of an absorbent article, the apparatus comprising:
a freely rotatable suction drum which includes, in a circumferential surface, a pattern plate which has a shape that is complementary to an outer shape of the absorbent body and in which a pattern hole that penetrates through in a thickness direction is formed, and a molding portion formed inside the pattern hole of the pattern plate, which forms the absorbent body by accumulating a material of the absorbent body; and
a supply device which includes a cover duct that covers a part of the circumferential surface of the suction drum, and which supplies the material of the absorbent body to the molding portion through the cover duct, wherein
the apparatus is provided with an air jetting mechanism which includes an air jetting port, and the air jetting port rotates with the rotation of the suction drum and jets out air toward an internal space of the cover duct, from both of or either one of the pattern plate and the molding portion.

According to the invention, the pattern plate includes an internal space facing portion which faces the internal space of the cover duct, in an external surface of the pattern plate which faces outward in a radial direction of the suction drum, and the air jetting port is provided in the internal space facing portion at positions adjacent to the pattern hole in the width direction (W). According to this configuration, the air is jetted out from the air jetting port provided in the internal space facing portion, whereby the material of the absorbent body which is to be accumulated in the vicinity of the air jetting port on the internal space facing portion of the pattern plate can be blown off. As a result, the material of the absorbent body accumulated on the internal space facing portion can be suppressed from forming the absorbent body together with the material of the absorbent body accumulated on the molding portion, whereby the outer shape of the absorbent body can be formed into a desired shape.

Still further, preferably, the air jetting port is provided in an internal surface of the pattern plate which defines the pattern hole. According to this configuration, an extremely thin air layer such as the one which is generated on an air hockey stand installed in an amusement center, and the like, can be formed in between the internal surface of the pattern plate and the accumulated material to form the absorbent body. Accordingly, the material of the absorbent body accumulated in the molding portion is pressed inwardly, and the material of the absorbent body accumulated on the molding portion is isolated from the external surface of the pattern plate, whereby forming the absorbent body together with the material of the absorbent body accumulated outside the pattern hole can be suppressed. As a result, an absorbent body having an outer shape along the outline shape of the pattern hole can be formed. Further, when extracting the absorbent body from the molding portion, the friction between the side portion of the absorbent body and the internal surface of the pattern plate can be reduced, whereby the extraction of the absorbent body from the molding portion becomes easier.

Still further, preferably, the air jetting mechanism jets out the air toward the internal space of the cover duct through the molding portion. According to this configuration, the basis weight of the absorbent body can be reduced at portions to which the air is blown, whereby the basis weight distribution of the absorbent body can be adjusted. As a result, an absorbent body of an absorbent article having a desired basis weight distribution can be manufactured.

Still further, preferably, the internal space of the cover duct is made to have a negative pressure by a suction of the suction drum, and the air jetting mechanism further includes an air intake port which is connected to the air jetting port and is released to an atmosphere. According to this configuration, the internal space of the cover duct is made to have a negative pressure, whereby air can be supplied from the atmosphere by the pressure differential, without using additional power. As a result, the apparatus can be simplified, in addition to being able to reduce the manufacturing cost of the absorbent body.

Still further, preferably, a plurality of the air jetting ports are provided, and one of the air jetting ports is not connected to another air jetting port, whose starting period and an ending period of facing the internal space of the cover duct, do not coincide with a starting period and an ending period of facing the internal space of the cover duct of the one of the air jetting ports, respectively. On the other hand, when another air jetting port being connected to one air jetting port which faces the internal space of the cover duct does not face the internal space of the cover duct, that is to say, is released to an atmosphere, the another air jetting port is to function as the air intake port. Then, the wind pressure and the wind speed of the air jetted out from the one air jetting port when the another air jetting port faces the internal space of the cover duct differ from those when the another air jetting port does not face the internal space of the cover duct. Accordingly, blowing off the air with a constant wind pressure and wind speed from the one air jetting port while facing the internal space of the cover duct is no longer possible. On the other hand, when one air jetting port is not connected to another air jetting port, whose starting period and an ending period of facing the internal space of the cover duct, do not coincide with a starting period and an ending period of facing the internal space of the cover duct of the one of the air jetting ports, respectively, as mentioned above, the air with a constant wind pressure and wind speed can always be blown from the one air jetting port, when the one air jetting port faces the internal space of the cover duct. As a result, an absorbent body of an absorbent article having a desired outer shape and basis weight distribution can be stably manufactured.

On the other hand, preferably, the air jetting mechanism includes an air supplying mechanism which supplies compressed air from an air intake port which is connected to the air jetting port toward the air jetting port. According to this configuration, the air with desired wind pressure and wind speed can be blown from the air jetting port. As a result, an absorbent body of an absorbent article having a desired outer shape and basis weight distribution can be manufactured.

Still further, preferably, a plurality of the air intake ports are provided, and the air supplying mechanism supplies the compressed air only to an air intake port which is connected to the air jetting port that faces the internal space of the cover duct, among the air intake ports. According to this configuration, the apparatus can be configured so that the air is jetted out only from the air jetting port facing the internal space of the cover duct, and the air is not jetted out from the air jetting port which does not face the internal space of the cover duct. As a result, the compressed air can be supplied only to the air jetting port which is required to jet out air, and the manufacturing cost of the absorbent body can be reduced.

### Advantageous Effects of Invention

The apparatus according to the present invention is provided with a jetting mechanism which includes an air jetting port that moves while rotating as the suction drum rotates and jets out air toward the internal space of the cover duct. Accordingly, the air can be blown to the external surface portions of the pattern plate at which the material of the absorbent body is accumulated, portions to which the material of the absorbent body is excessively supplied, and the like. As a result, according to the present invention, an apparatus capable of manufacturing an absorbent body having a desired outer shape and basis weight distribution, even in a case of manufacturing an absorbent body of an absorbent article having a complicated outer shape, can be provided.

### Brief Description of Drawing

[FIG. 1] FIG. 1 is a schematic diagram of an apparatus to manufacture an absorbent body of an absorbent article, according to a first embodiment of the present invention.
[FIG. 2] FIG. 2 is a planar view of a pattern plate of the apparatus to manufacture the absorbent body of the absorbent article, according to the first embodiment.
[FIG. 3] FIG. 3 is a sectional view taken along line III-III of FIG. 1.
[FIG. 4] FIG. 4 is a sectional view taken along line IV-IV of FIG. 2.
[FIG. 5] FIG. 5 is a schematic sectional view of an apparatus to manufacture an absorbent body of an absorbent article, according to a second embodiment of the present invention.
[FIG. 6] FIG. 6 is a schematic sectional view of an apparatus to manufacture an absorbent body of an absorbent article, according to a third embodiment of the present invention.
[FIG. 7] FIG. 7 is a schematic sectional view of an apparatus to manufacture an absorbent body of an absorbent article, according to a fourth embodiment of the present invention.

### Description of Embodiments

The present invention is described in detail with reference to the above mentioned drawings. Incidentally, it should be noted that these drawings may not be depicted in the same size, scale, shape as those of the actual configuration elements, in order to facilitate understanding of the present invention and to simplify the description of the drawings.

### (First Embodiment)

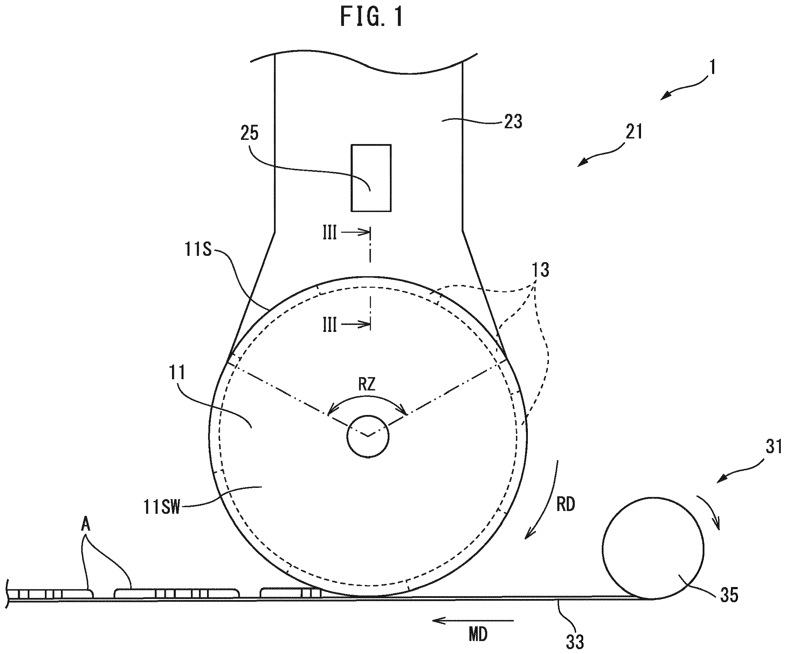

Hereinbelow, the apparatus to manufacture an absorbent body of an absorbent article according to the first embodiment of the present invention is described with reference to FIGS. 1-4. The apparatus 1 according to the first embodiment includes a freely rotatable suction drum 11, and a supply device 21 that includes a cover duct 23 which is a part of the circumferential surface 11S of the suction drum 11 and, in the first embodiment, covers the upper portion of the circumferential surface 11S. The apparatus 1 according to the first embodiment further includes an absorbent body conveying mechanism 31 which conveys the formed absorbent body A to a downstream process.

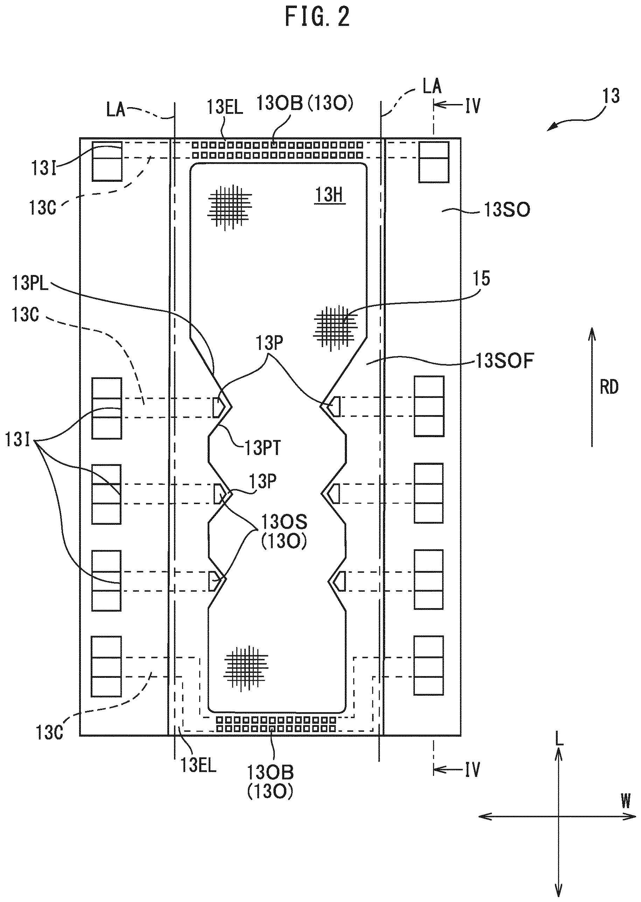

In the first embodiment, as shown in FIGS. 1 and 2, the suction drum 11 includes, in the circumferential surface, a plurality of pattern plates 13 in each of which a pattern hole 13H is formed, and a molding portion 15 formed in each of the pattern holes 13H. A rotation driving device (which is not shown), such as a servo motor, and the like, is connected to a shaft portion of the suction drum 11, so that the suction drum 11 can be rotationally driven at a desired peripheral speed.

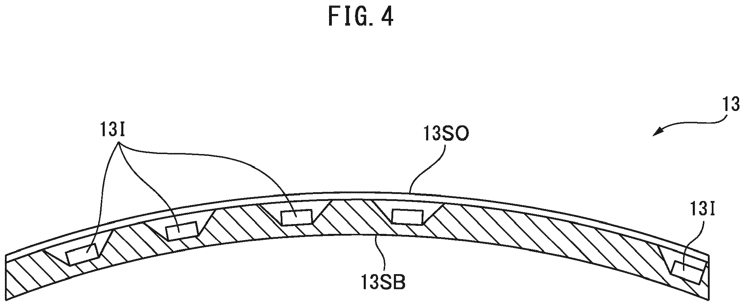

As shown in FIG. 4, the pattern plate 13 is a metal plate which is curved with the curvature of the circumferential surface 11S of the suction drum 11 so as to form the circumferential surface 11S of the suction drum 11, and has a certain thickness. The pattern hole 13H of the pattern plate 13 has a planar shape that is complementary to the outer shape of the absorbent body A to be manufactured, and penetrates through in a thickness direction. In the first embodiment, the pattern plate 13 is attached to a flange portion 11F which extends inward in the width direction of the suction drum 11 from the internal surface of the side wall 11SW of the suction drum 11, by a publicly known fastening means (which is not shown), such as bolts, and the like.

The molding portion 15 which is formed in the pattern hole 13H of the pattern plate 13 forms the absorbent body A by accumulating the material of the absorbent body thereon, and in the first embodiment, is formed by a metal mesh (a net). Incidentally, in FIG. 2, only a part of the mesh is shown in the molding portion 15 for the sake of clarity of the drawing, however, the molding portion 15 is formed entirely in the pattern hole 13H.

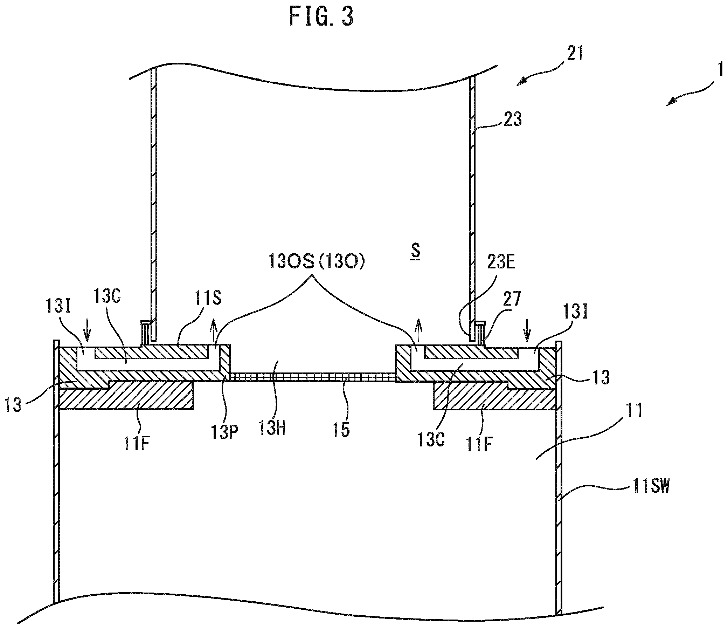

Further, an air discharging device (which is not shown) for discharging the air inside the suction drum 11, such as a blower, a vacuum pump, and the like, is connected to the suction drum 11, and by operating the air discharging device, the inside of the suction drum 11 is made to have a negative pressure. Accordingly, in the first embodiment, the circumferential surface 11S of the suction drum 11 is configured to suck the air toward the inside of the suction drum 11 from the internal space S of the cover duct 23, through the pattern hole 13H of the pattern plate 13, in the circumferential direction region RZ which is covered by the cover duct 23. Further, as shown in FIG. 3, the circumferential surface 11S of the suction drum 11 and the lower end 23E of the cover duct 23 are close to each other, and the abutting member 27 which is, for example, a metal brush, felt, and the like, is provided at the lower end 23E of the cover duct 23, and is arranged so as to extend toward the circumferential surface 11S of the suction drum 11 and have the tip thereof abut against the circumferential surface 11S of the suction drum 11. Accordingly, a constant airtightness of the internal space S of the cover duct 23 is ensured. As a result, in the first embodiment, by the suction action by the air discharging device of the suction drum 11, the internal space S of the cover duct 23 can be made to have negative pressure, and the supplied material of the absorbent body can be sucked against the molding portion 15 and be accumulated thereon. Incidentally, in the present description, "an internal space of a cover duct" means the space defined by the circumferential surface 11S of the suction drum 11, which is defined by the pattern plate 13 and the molding portion 15, and by the internal wall surface of the cover duct 23.

In the apparatus 1 according to the first embodiment, fibers and super absorbent polymers (SAP) which are the material of the absorbent body are supplied to the circumferential surface 11S of the suction drum 11, and especially to the molding portion 15, in the circumferential direction region RZ which is covered by the cover duct 23, through the cover duct 23. The fibers are supplied in an amount corresponding to the basis weight of the absorbent body A to be manufactured, in a state of being opened by an opening device (which is not shown), such as a carding machine, and the like, placed in the upstream of the cover duct 23 (upper direction in FIG. 1). Further, a predetermined amount of the super absorbent polymer material is supplied to the inside of the cover duct 23 from the super absorbent polymer material supplying portion 25 which is opened inside the cover duct 23.

Further, in the first embodiment, in the pattern plate 13, a plurality of air jetting ports 13O in the external surface 13SO of the pattern plate 13 which forms the circumferential surface 11S of the suction drum 11, and an air intake port 13I which is connected to each of the air jetting ports 13O through the air vent passage 13C are provided. As shown in FIG. 3, each of the air jetting ports 13O faces outward in the radial direction of the suction drum 11, and is capable of jetting out the air in the radial direction of the suction drum 11. On the other hand, the each of the air intake ports 13I is released to the atmosphere. Further, in the first embodiment, each of the air vent passages 13C extends at a substantially constant position in the thickness direction of the pattern plate 13, and is formed such that there is no seam therethrough, i.e., the cross sectional area thereof does not radically enlarge or radically shrink.

In the first embodiment, each of the air intake ports 131 is provided at the end portions of the pattern plate 13 in the width direction W. Further, as shown in FIG. 2, the air jetting ports 13O include a plurality of external surface air jetting ports 130S which are provided at positions adjacent to the pattern hole 13H in the width direction W, and border air jetting ports 130B which are provided at positions adjacent to the pattern hole 13H in the longitudinal direction L, at both of the longitudinal direction end portions 13EL of the pattern hole 13H. To be more specific, each of the external surface air jetting ports 13OS is provided at the protruded portion 13P which protrudes with respect to the pattern hole 13H toward the center in the width direction W of the pattern plate 13. Each of the border air jetting ports 13OB is provided across the substantially whole length of the end portion along the end portion in the longitudinal direction L of the pattern plate 13H. Incidentally, FIG. 2 seems to describe a number of small holes as the border air jetting ports 13OB, however, these are actually partially blocking the holes to suppress the fibers from entering the border air jetting ports 13OB. Accordingly, in the first embodiment, one border air jetting port 13OB is provided for each of the both longitudinal direction end portions 13EL of the pattern plate 13.

The absorbent body conveying mechanism 31 receives the absorbent body A formed in the molding portion 15 from the suction drum 11, and conveys the absorbent body A to the downstream process. In the first embodiment, the absorbent body conveying mechanism 31 includes a carrier sheet feeding portion 35 for feeding out a carrier sheet 33 which may be a nonwoven fabric, and the like, in the conveying direction MD, and a conveying device (which is not shown), such as a belt conveyer for conveying the fed carrier sheet 33 in the conveying direction MD, and the like. Incidentally, the suction drum 11 may include a mechanism that blows air to the molding portion 15 from inside of the suction drum 11, so as to extract the absorbent body A from the pattern hole 13H, when delivering the absorbent body A from the suction drum 11 to the carrier sheet 33.

Hereinbelow, the operation and the effect of the apparatus 1 according to the first embodiment are described.
(1) In the first embodiment, in the pattern plate 13, the protruded portions 13P which protrude toward the pattern hole 13H in the center in the width direction W of the pattern plate 13 are formed, so as to form the absorbent body A into a desired shape. Since these protruded portions 13P are protruded with respect to the pattern hole 13H, when the external surface air jetting ports 13OS are not provided, it is easier for the material of the absorbent body to be accumulated thereon compared to other portions of the external surface 13SO of the pattern plate 13. Then, the material of the absorbent body accumulated on these protruded portions 13P may form the absorbent body A together with the material of the absorbent body accumulated on the molding portion 15, whereby the absorbent body A may not be formed into a desired shape outlined by the pattern hole 13H. In order to suppress such situation, in the first embodiment, the external surface air jetting ports 130S are provided in the protruded portions 13P, as described above. Accordingly, air is jetted out from the external surface air jetting ports 13OS, so as to blow off the material of the absorbent body, whereby the accumulation of the material of the absorbent body on the protruded portions 13P can be prevented, and the absorbent body A can be formed only by the material of the absorbent body accumulated on the molding portion 15. As a result, the absorbent body A having the desired shape can be formed by the apparatus 1 according to the first embodiment.
(2) Further, in the first embodiment, the front edges 13PL and the rear edges 13PT of the protruded portion 13P extending in a direction inclined with respect to the rotation direction RD of the suction drum 11, as formed by the protruded portion 13P are formed. When the external surface air jetting ports 13OS are not provided, since the pattern plate 13 moves while rotating as the suction drum 11 rotates, the material of the absorbent body is supplied in a concentrated manner to the portions of the molding portion 15 in the vicinity of the front edges 13PL of the protruded portion 13P, and on the contrary, only a little amount of the material of the absorbent body is supplied to the portions of the molding portion 15 in the vicinity of the rear edges 13PT of the protruded portion 13P, due to the shape of the protruded portions 13P. As a result, the basis weight of the absorbent body A may be uneven from portions to portions, whereby there may be cases where the function of the absorbent body A cannot be demonstrated in the desired manner. In order to suppress such situation, in the first embodiment, the external surface air jetting ports 130S are provided in the protruded portions 13P, as described above. Accordingly, by temporarily blowing off the material of the absorbent body supplied to the molding portion 15, in the front edges 13PL side and in the vicinity of the rear edges 13PT, the supply distribution of the material of the absorbent body can be changed intentionally. As a result, the absorbent body A having the desired basis weight distribution even in the front edges 13PL side and in the vicinity of the rear edges 13PT can be manufactured.
(3) In the apparatus 1 according to the first embodiment, one absorbent body A is manufactured per one pattern plate 13, and each of the absorbent bodies A is formed as a separate body. Accordingly, in the longitudinal direction end portions 13EL of the pattern plate 13, not the pattern hole 13H but the external surface 13SO of the pattern plate 13 is formed. Consequently, when the border air jetting ports 13OB as described above are not provided, since the center portion in the width direction W in the longitudinal direction end portions 13EL of the pattern plate 13 is located at the portion extended in the longitudinal direction L of the pattern plate 13 from the molding portion 15, it is easier for the material of the absorbent body to be accumulated thereon compared to other portions of the external surface 13SO of the pattern plate 13. As a result, there may be cases where the outer shape at the longitudinal direction end portions of the absorbent body A is not formed in the desired manner, and in extreme cases, two absorbent bodies A may be connected to each other. In order to suppress such situation, in the first embodiment, the border air jetting ports 13OB are provided at the longitudinal direction end portions 13EL of the pattern plate 13, as described above, whereby the material of the absorbent body can be suppressed from being accumulated at the longitudinal direction end portions 13EL of the pattern plate 13. As a result, the absorbent body A having the desired outer shape at the longitudinal direction end portion of the absorbent body A can be formed.
(4) In the first embodiment, as described above, the internal space S of the cover duct 23 is made to have a negative pressure by the suction of the air discharging device (which is not shown) connected to the suction drum 11, and the air jetting ports 13O are connected to the air intake ports 13I which are released to the atmosphere, through the air vent passages 13C. Accordingly, the air can be supplied to the internal space S of the cover duct 23 from the atmosphere, by the pressure differential between the internal space S of the cover duct 23 and the atmosphere, without using additional power. As a result, the apparatus 1 can be simplified, in addition to being able to reduce the manufacturing cost of the absorbent body A. Incidentally, in the apparatus 1 according to the first embodiment, since the air is jetted out from the air jetting ports 13O in accordance with the mechanism as described above, the air jetting mechanism is configured by the air discharging device (which is not shown) connected to the suction drum 11, the air intake ports 131, the air vent passages 13C, and the air jetting ports 130.
(5) In the first embodiment, each of the air vent passages 13C extends at the substantially constant position in the thickness direction of the pattern plate 13, and is formed such that there is no seam therethrough, and the cross sectional area thereof does not radically enlarge or radically shrink, as described above. Each of the air vent passages 13C extends at the substantially constant position in the thickness direction of the pattern plate 13, whereby each of the air vent passages 13C has substantially no variation in the thickness direction of the pattern plate 13 inside the pattern plate 13. As a result, the pattern plate 13 can be thinly formed, whereby the manufacturing time and the manufacturing cost of the pattern plate 13 can be reduced. Further, each of the air vent passages 13C has no seam therethrough, whereby radical enlargement and radical shrinkage of the cross section area of each of the air vent passages 13C are not generated, and the pressure loss of the air passing through the air vent passages 13C can be suppressed, and hence the decrease in the energy efficiency of the air jetted out from the air jetting ports 13O can be suppressed.

In the first embodiment, the external surface air jetting ports 13OS are provided in the protruded portions 13P, however, the external surface air jetting ports 13OS may be provided in other portions. In other words, the external surface air jetting ports 13OS may be provided at any portions of the internal space facing portion 13SOF which faces the internal space S of the cover duct 23, among the external surface 13SO of the pattern plate 13. Incidentally, it should be noted that the internal space facing portion 13SOF refers to the portion of the external surface 13SO of the pattern plate 13 which intermittently faces the internal space S of the cover duct 23, as the suction drum 11 moves while rotating, and does not always face the internal space S of the cover duct 23. More specifically, in the first embodiment, the internal space facing portion 13SOF refers to the region between the contact lines LA which are the locus of the portions at which the tip of the abutting member 27 of the lower end 23E of the cover duct 23 abuts against the external surface 13SO of the pattern plate 13.

In the first embodiment, as shown in FIG. 2, each of the external surface air jetting ports 13OS are connected to one air intake port 131, each of the border air jetting ports 13OB is connected to the two air intake ports 13I positioned at both sides in the width direction W, and the air jetting ports 13O are not connected to each other. However, the present invention is not limited to this embodiment. For example, two air vent passages 13C may be connected to each other, whereby two air jetting ports 13O may be connected to each other. However, the air jetting ports 13O are preferably not connected to each other, as described in the first embodiment. To be more specific, one air jetting port 13O is preferably not connected to another air jetting port 13O whose starting period and ending period of facing the internal space S of the cover duct 23, do not coincide with a starting period and an ending period of facing the internal space S of the cover duct 23 of the one air jetting port 13O, respectively. On the other hand, when one air jetting port 13O is connected to another air jetting port 13O whose starting period and ending period of facing the internal space S of the cover duct 23, do not coincide with a starting period and an ending period of facing the internal space S of the cover duct 23 of the one air jetting port 13O, respectively, a moment at which the one air jetting port 13O faces the internal space S of the cover duct 23 and the another air jetting port 13O does not face the internal space S of the cover duct 23, i.e., the another air jetting port 13O is released to the atmosphere is present. At this moment, the another air jetting port 13O is to function as the air intake port of the one air jetting port 13O. Then, the wind pressure and the wind speed of the air jetted out from the one air jetting port 13O when the another air jetting port 13O faces the internal space S of the cover duct 23 differ from those when the another air jetting port 13O does not face the internal space S of the cover duct 23. On the other hand, when one air jetting port 13O is not connected to another air jetting port 13O whose starting period and ending period of facing the internal space S of the cover duct 23, do not coincide with a starting period and an ending period of facing the internal space S of the cover duct 23 of the one air jetting port 13O, respectively, as mentioned above, the air with a constant wind pressure and wind speed can always be blown from the one air jetting port 13O, when the one air jetting port 13O faces the internal space S of the cover duct 23. As a result, the absorbent body A of an absorbent article having the desired outer shape and basis weight distribution can be stably manufactured.

Incidentally, in the first embodiment, the opening cross section of the cover duct 23 which opens to the circumferential surface 11S of the suction drum 11 is substantially rectangular. Accordingly, in order for the two air jetting ports 13O to have starting periods and ending periods of facing the internal space S of the cover duct 23 that coincide with each other, the positions in the width direction W of the air jetting ports 13O should match. Accordingly, in the first embodiment, two air jetting ports 13O, the positions of which in the longitudinal direction L of the pattern plate 13 are different from each other, are preferably not connected to each other.

In the first embodiment, one external surface air jetting port 13OS is provided for each of the protruded portions 13P, however, the present invention is not limited to this embodiment. In the present invention, in order to prevent the material of the absorbent body from being accumulated on the external surface 13SO of the pattern plate 13, or to obtain the desired supply distribution of the material of the absorbent body, the shape, the number, the position of the external surface air jetting ports 13OS, and the wind pressure and wind speed of the air jetted out from the external surface air jetting ports 13OS, may be determined by, for example, an experiment, and the like. For example, in another embodiment, in each of the protruded portions 13P, a plurality of air jetting ports which are smaller than the external surface air jetting ports 13OS are provided along the front edges 13PL side and the rear edges 13PT of the protruded portions 13P. Further, in still another embodiment, an elongated air jetting port is provided along the front edges 13PL and the rear edges 13PT of the protruded portions 13P, in the pattern plate 13.

Further, in the first embodiment, the air jetting port 13O faces outward in the radial direction of the suction drum 11, and jets out the air in the radial direction of the suction drum 11. However, in the present invention, in order to obtain the absorbent body A having the desired outer shape and basis weight distribution, the air jetting port 13O may be formed so that the air jetting port 13O faces in any direction and jets out the air therefrom.

In the first embodiment, the molding portion 15 is flat, however, the molding portion 15 may have protrusions and depressions in the thickness direction. Further, the molding portion 15 is a metal mesh, however, in the present invention, the molding portion may not be metallic, and may alternatively be a plate having a plurality of small holes.

### (Second Embodiment)

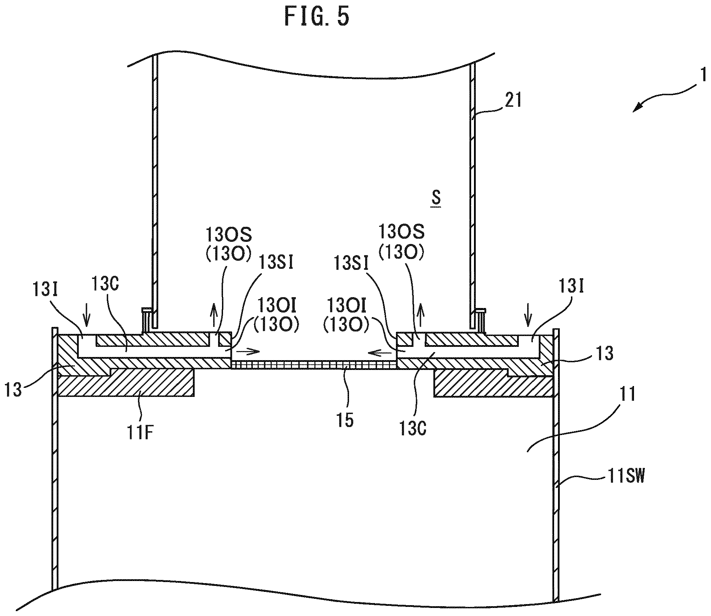

Hereinbelow, with reference to FIG. 5 which corresponds to FIG. 3, the apparatus to manufacture an absorbent body of an absorbent article according to the second embodiment of the present invention will be described. In the second embodiment, the differences from the first embodiment will be mainly described. Further, it should be noted that the configurations in the first embodiment other than those different from the second embodiment are applicable to the second embodiment, and that a person skilled in the art can arbitrarily combine these configurations within obvious ranges.

In the second embodiment, in the pattern plate 13, the air vent passage 13C which connects the external surface air jetting port 13OS and the air intake port 13I is branched off, and the internal surface air jetting port 13OI which is the air jetting port 13O being opened from the internal surface 13SI that defines the pattern hole 13H is formed.

By being formed with the internal surface air jetting port 13OI, the air can be blown to the material of the absorbent body which is accumulated on the molding portion 15, from the side. According to this configuration, an extremely thin air layer such as the one which is generated on an air hockey stand installed in an amusement center, and the like can be formed in between the internal surface 13SI of the pattern plate 13 and the material of the absorbent body accumulated on the molding portion 15. Accordingly, the material of the absorbent body accumulated on the molding portion 15 is isolated from the external surface 13SO of the pattern plate 13, whereby forming the absorbent body A together with the material of the absorbent body accumulated outside the pattern hole 13H can be suppressed. As a result, the absorbent body A having an outer shape along the outline shape of the pattern hole 13H can be formed. Further, when extracting the absorbent body A from the molding portion 15, the friction between the side portion of the absorbent body A and the internal surface SI of the pattern plate 13 can be reduced, whereby the extraction of the absorbent body A from the molding portion 15 becomes easier.

In the second embodiment, the air vent passage 13C which is connected to one air intake port 131 is branched off, and the air supplied form the air intake port 13I jets out from both the internal surface air jetting port 13OI and the external surface air jetting port 13OS, however, the present invention is not limited to such configuration. For example, in an apparatus according to another embodiment, the external surface air jetting port 13OS is not provided, and only the internal surface air jetting port 13OI is provided, and one air intake port 13I is provided for one internal surface air jetting port 13OI. Further, in still another embodiment, separate air intake port 13I and air vent passage 13C are provided to supply air to the internal surface air jetting port 13OI and the external surface air jetting port 13OS, and the air is supplied to the internal surface air jetting port 1301 and the external surface air jetting port 13OS from each of the air intake ports 131 through each of the air vent passages 13C.

### (Third Embodiment)

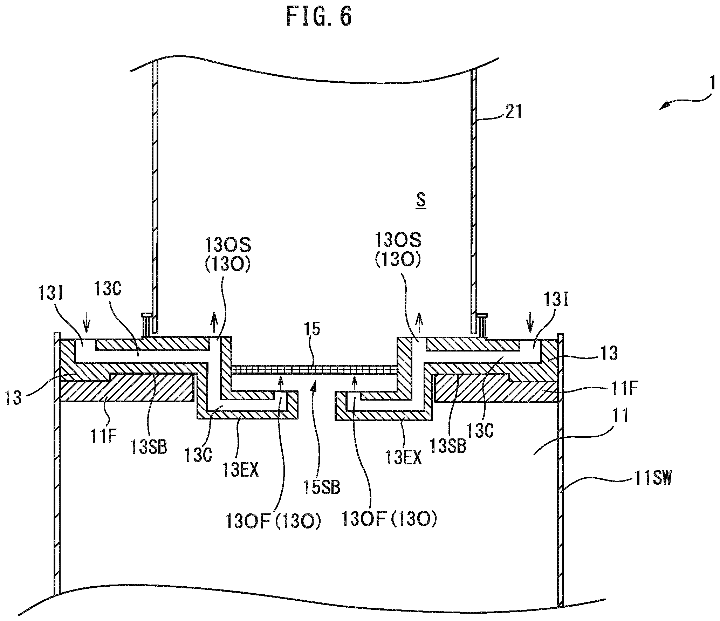

Hereinbelow, with reference to FIG. 6 which corresponds to FIG. 3, the apparatus to manufacture an absorbent body of an absorbent article according to the third embodiment of the present invention will be described. In the third embodiment, the differences from the first embodiment will be mainly described. Further, it should be noted that the configurations in the first embodiment other than those different from the third embodiment are applicable to the third embodiment, and that a person skilled in the art can arbitrarily combine these configurations within obvious ranges.

In the third embodiment, the extended portion 13EX, which protrudes inwardly in the radial direction of the suction drum 11 from the back surface 13SB, i.e., opposite to the external surface 13SO of the pattern plate 13, and extends to the position which faces the back surface 15SB, i.e., in the opposite side from the side at which the material of the absorbent body is accumulated in the molding portion 15, is additionally formed for the pattern plate 13. At the tip portion of the extended portion 13EX, the molding portion air jetting port 13OF is provided in order to blow air toward the back surface 15SB of the molding portion 15, and to blow the air to the internal space S of the cover duct 23 from the molding portion 15.

As shown in FIG. 6, the air vent passage 13C which connects the external surface air jetting port 13OS and the air intake port 13I is branched off, and the air intake port 13I and the molding portion air jetting port 130F are connected to each other, whereby the air inhaled from the air intake port 131 can be jetted out from the molding portion air jetting port 13OF.

In the third embodiment, the air is jetted out toward the internal space S of the cover duct 23 through the molding portion 15 from the molding portion air jetting port 13OF, whereby the material of the absorbent body accumulated on the molding portion 15 can be blown off and the accumulation amount thereof can be reduced, at the portion where the air is blown. As a result, the absorbent body A having the desired basis weight can be manufactured. Alternatively, the air can be blown to the portions of the absorbent body A where the basis weight thereof is unintentionally increased in the apparatus 1 according to the first embodiment, from the molding portion air jetting port 13OF, and the basis weight thereof can be equalized with that of the other portions, whereby the absorbent body A having an equalized basis weight distribution can be manufactured.

In FIG. 6, two extended portions 13EX and two molding portion air jetting ports 13OF are described, however, the present invention is not limited to this embodiment. For example, the number of the extended portions 13EX and the molding portion air jetting ports 13OF, and the portion to which the air is blown from the molding portion air jetting ports 13OF can be arbitrarily set. Further, the shape of the extended portion 13EX can be arbitrarily determined as long as the molding portion air jetting port 13OF can be disposed as desired and the accumulation of the material of the absorbent body in the molding portion 15 is not inhibited.

In the third embodiment, the extended portion 13EX is provided in the pattern plate 13, and the molding portion air jetting port 13OF is provided at the tip thereof, however, the present invention is not limited to this embodiment, as long as a mechanism in order to blow the air to the internal space S of the cover duct 23 from the molding portion 15 is provided. For example, the side wall 11SW of the suction drum 11 may be provided with a penetrated hole, a hose which a nozzle is attached at the tip thereof may be connected to the penetrated hole, and the nozzle opening may be fixed facing toward the molding portion 15 so that the nozzle can move while rotating as the suction drum 11 rotates, whereby the air may be blown from the nozzle opening. In this configuration, the nozzle opening corresponds to the molding portion air jetting port 13OF in the third embodiment.

### (Fourth Embodiment)

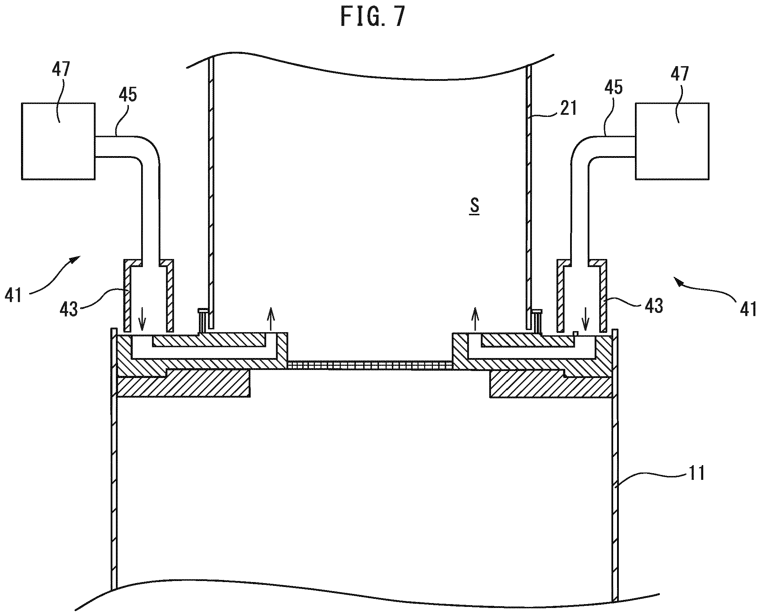

Hereinbelow, with reference to FIG. 7 which corresponds to FIG. 3, the apparatus to manufacture an absorbent body of an absorbent article according to the fourth embodiment of the present invention will be described. In the fourth embodiment, the differences from the first embodiment will be mainly described. Further, it should be noted that the configurations in the first embodiment other than those different from the fourth embodiment are applicable to the fourth embodiment, and that a person skilled in the art can arbitrarily combine these configurations within obvious ranges.

The air supplying mechanism 41 which supplies compressed air from the air intake port 13I toward the air jetting port 13O is provided in the apparatus 1 according to the fourth embodiment. As shown in FIG. 7, the air supplying mechanism 41 is configured by including the air supplying cover 43 which covers the air intake port 13I, and the compressor 47 which supplies the compressed air to the inside of the air supplying cover 43 through the duct 45.

The air supplying cover 43 is hermetically sealed with respect to the outside to some extent, so that the compressed air supplied from the compressor 47 can flow into the air intake port 131 efficiently, whereby a constant airtightness of the inside of the air supplying cover 43 is ensured. In order to ensure the constant airtightness of the inside of the air supplying cover 43, an abutting member which abuts the external surface 13SO of the pattern plate 13 may be provided at the lower end of the air supplying cover 43, in the same manner as the cover duct 23.

According to this configuration, the compressed air with desired wind pressure and wind speed can be blown from the air jetting port 13O. As a result, an absorbent body of an absorbent article having a desired outer shape and basis weight distribution can be manufactured.

Further, the air supplying mechanism 41 preferably supplies the compressed air only to the air intake port 13I which is connected to the air jetting port 13O that faces the internal space S of the cover duct 23, among the air intake ports 13I. To be more specific, in the case of the fourth embodiment, the air supplying cover 43 preferably covers the air intake port 131 in the above described circumferential direction region RZ, by the approximately same range as the cover duct 23 covers the circumferential surface 11S of the suction drum 11 in the rotation direction RD of the suction drum 11. According to this configuration, the apparatus can be configured so that the air is jetted out only from the air jetting port 13O facing the internal space S of the cover duct 23, and the air is not jetted out from the air jetting port 13O which does not face the internal space S of the cover duct 23. As a result, the compressed air can be supplied only to the air jetting port 13O which is required to jet out air, and the manufacturing cost of the absorbent body A can be reduced.

In the above-described embodiment, the pattern plate 13 is made of a metal, and more specifically, the pattern plate 13 is formed by a plurality of metal plates being laminated and then sintered and joined so as to be integrated. However, in the present invention, the forming method of the pattern plate 13 is not limited to this, and any manufacturing method thereof can be used. For example, in another embodiment, the pattern plate is made of photocurable resin and is formed by a 3D printer.

Incidentally, all the features which can be understood by those skilled in the art from the description of the specification, drawings and the claims can be applied independently or can be applied in optional combination with another one or a plurality of features disclosed herein to be bound together, as long as such features are explicitly excluded or its technical aspect becomes an impossible or a meaningless combination, even when such features are described only in combination with another specific feature in this specification.

For example, an apparatus according to another embodiment includes the suction drum 11 in the circumferential surface, which includes the pattern plate 13 in which both of the internal surface air jetting port 1301 that is shown in the second embodiment and the molding portion air jetting port 130F that is shown in the third embodiment are provided.

Further, the air supplying mechanism 41 which supplies the compressed air toward the air jetting port 13O from the air intake port 13I, that is shown in the fourth embodiment may be provided in the apparatus 1 according to the second and the third embodiments.

### Reference Signs List

1 apparatus (to manufacture an absorbent body of an absorbent article)
11 suction drum
11S circumferential surface
13 pattern plate
13C air vent passage (air jetting mechanism)
13H pattern hole
131 air intake port (air jetting mechanism)
130 air jetting port (air jetting mechanism)
13SO external surface
15 molding portion
21 supply device
23 cover duct
41 air supplying mechanism
A absorbent body
S internal space (of a cover duct)

## Claims

1. An apparatus (1) to manufacture an absorbent body (A) of an absorbent article, the apparatus (1) comprising:
a freely rotatable suction drum (11) which includes, in a circumferential surface (11 S), a pattern plate (13) which has a shape that is complementary to an outer shape of the absorbent body (A) and in which a pattern hole (13H) that penetrates through in a thickness direction is formed, and a molding portion (15) formed inside the pattern hole (13H) of the pattern plate (13), which forms the absorbent body (A) by accumulating a material of the absorbent body (A); and
a supply device (21) which includes a cover duct (23) that covers a part of the circumferential surface (11S) of the suction drum (11), and which supplies the material of the absorbent body (A) to the molding portion (15) through the cover duct (23), wherein
the apparatus (1) is provided with an air jetting mechanism which includes an air jetting port (130), and the air jetting port (130) rotates with the rotation of the suction drum (11) and jets out air toward an internal space (S) of the cover duct (23), from both of or either one of the pattern plate (13) and the molding portion (15),
the pattern plate (13) includes an internal space facing portion (13SOF) which faces the internal space (S) of the cover duct (23), in an external surface of the pattern plate (13) which faces outward in a radial direction of the suction drum (11),
the air jetting port (13O) is provided in the internal space facing portion (13SOF), and
the air jetting port (13O) is provided at positions adjacent to the pattern hole (13H) in the width direction (W).

2. The apparatus (1) according to claim 1, wherein the air jetting mechanism jets out the air toward the internal space (S) of the cover duct (23) through the molding portion (15).

3. The apparatus (1) according to any one of claims 1 to 2, wherein the internal space (S) of the cover duct (23) is made to have a negative pressure by a suction of the suction drum (11), and
the air jetting mechanism further includes an air intake port (13I) which is connected to the air jetting port (13O) and is released to an atmosphere.

4. The apparatus according to claim 3, wherein a plurality of the air jetting ports (13O) are provided, and
one of the air jetting ports (13O) is not connected to another air jetting port (13O), whose starting period and an ending period of facing the internal space (S) of the cover duct (23), do not coincide with a starting period and an ending period of facing the internal space (S) of the cover duct (23) of the one of the air jetting ports (13O), respectively.

5. The apparatus (1) according to any one of claims 1 to 2, wherein the air jetting mechanism includes an air supplying mechanism (41) which supplies compressed air from an air intake port (13I) which is connected to the air jetting port (13O) toward the air jetting port (13O).

6. The apparatus (1) according to claim 5, wherein a plurality of the air intake ports (13I) are provided, and
the air supplying mechanism (41) supplies the compressed air only to an air intake port (13I) which is connected to the air jetting port (13O) that faces the internal space (S) of the cover duct (23), among the air intake ports (13I).

## Patentansprüche

1. Vorrichtung (1) zum Herstellen eines Absorptionskörpers (A) eines Absorptionsartikels, wobei die Vorrichtung (1) aufweist:
eine frei drehbare Saugtrommel (11), die in einer Umfangsfläche (11S) eine Musterplatte (13) aufweist, die eine Form, die zu einer Außenform des Saugkörpers (A) komplementär ist und in der ein Musterloch (13H), das in einer Dickenrichtung durchdringt, gebildet worden ist, und einen Formabschnitt (15) aufweist, der innerhalb des Musterlochs (13H) der Musterplatte (13) gebildet worden ist, der den Absorptionskörper (A) durch ein Ansammeln eines Materials des Absorptionskörpers (A) bildet, und
eine Zuführvorrichtung (21), die einen Abdeckkanal (23) aufweist, der einen Teil der Umfangsfläche (11S) der Saugtrommel (11) abdeckt und der das Material des Absorptionskörpers (A) dem Formabschnitt (15) durch den Abdeckkanal (23) zuführt, wobei
die Vorrichtung (1) mit einem Luftstrahlmechanismus versehen ist, der eine Luftausstoßöffnung (13O) aufweist, und wobei die Luftausstoßöffnung (13O) sich mit der Drehung der Saugtrommel (11) dreht und Luft in Richtung auf einen Innenraum (S) des Abdeckkanals (23) von der Musterplatte (13) und / oder dem Formabschnitt (15) ausstößt, wobei
die Musterplatte (13) einen dem Innenraum zugewandten Abschnitt (13SOF), der dem Innenraum (S) des Abdeckungskanals (23) zugewandt ist, in einer äußeren Oberfläche der Musterplatte (13) enthält, die in einer radialen Richtung der Saugtrommel (11) in Richtung nach außen weist, wobei
die Luftausstoßöffnung (13O) in dem Abschnitt (13SOF) vorgesehen ist, der dem Innenraum zugewandt ist, und wobei
die Luftausstoßöffnung (13O) an Positionen vorgesehen ist, die in der Breitenrichtung (W) an das Musterloch (13H) angrenzen.

2. Vorrichtung (1) nach Anspruch 1, wobei der Luftstrahlmechanismus die Luft durch den Formabschnitt (15) in Richtung auf den Innenraum (S) des Abdeckungskanals (23) ausstößt.

3. Vorrichtung (1) nach einem der Ansprüche 1 bis 2, wobei der Innenraum (S) des Abdeckkanals (23) durch ein Ansaugen der Saugtrommel (11) auf Unterdruck gebracht worden ist, und
der Luftstrahlmechanismus ferner eine Lufteinlassöffnung (13I) aufweist, die mit der Luftausstoßöffnung (13O) verbunden ist und an eine Atmosphäre abgegeben wird.

4. Vorrichtung nach Anspruch 3, wobei eine Vielzahl an Luftausstoßöffnungen (13O) vorgesehen sind und
eine der Luftausstoßöffnungen (13O) nicht mit einer anderen Luftausstoßöffnung (13O) verbunden ist, von der eine Startperiode und eine Endperiode, die dem Innenraum (S) des Abdeckkanals (23) zugewandt sind, nicht mit einer Startperiode und eine Endperiode übereinstimmen, in der der Innenraum (S) des Abdeckungskanals (23) der einen der Luftausstoßöffnungen (13O) zugewandt ist.

5. Vorrichtung (1) nach einem der Ansprüche 1 bis 2, wobei der Luftstrahlmechanismus einen Luftzufuhrmechanismus (41) aufweist, der Druckluft von einer Lufteinlassöffnung (13I) zuführt, die mit der Luftausstoßöffnung (13O) in Richtung auf die Luftausstoßöffnung (13O) verbunden ist.

6. Vorrichtung (1) nach Anspruch 5, wobei eine Vielzahl an Lufteinlassöffnungen (13I) vorgesehen sind und
der Luftzufuhrmechanismus (41) die Druckluft unter den Lufteinlassöffnungen (13I) nur einer Lufteinlassöffnung (13I) zuführt, die mit der Luftausstoßöffnung (13O) verbunden ist, die dem Innenraum (S) des Abdeckungskanals (23) gegenüberliegt.

## Revendications

1. Appareil (1) pour fabriquer un corps absorbant (A) d'un article absorbant, l'appareil (1) comprenant :
un tambour d'aspiration librement rotatif (11) qui inclut, dans une surface circonférentielle (11S), une plaque de motif (13) qui a une forme qui est complémentaire à une forme extérieure du corps absorbant (A) et dans laquelle est formé un trou de motif (13H) qui traverse dans une direction d'épaisseur, et une portion de moulage (15) formée à l'intérieur du trou de motif (13H) de la plaque de motif (13), qui forme le corps absorbant (A) en accumulant un matériau du corps absorbant (A) ; et
un dispositif d'alimentation (21) qui inclut un conduit de couverture (23) qui couvre une partie de la surface circonférentielle (11 S) du tambour d'aspiration (11), et qui apporte le matériau du corps absorbant (A) à la portion de moulage (15) par le biais du conduit de couverture (23), dans lequel
l'appareil (1) est pourvu d'un mécanisme d'éjection d'air qui inclut un orifice d'éjection d'air (13O), et l'orifice d'éjection d'air (13O) tourne avec la rotation du tambour d'aspiration (11) et éjecte de l'air vers un espace interne (S) du conduit de couverture (23), depuis les deux ou l'une parmi la plaque de motif (13) et la portion de moulage (15),
la plaque de motif (13) inclut une portion faisant face à l'espace interne (13SOF) qui fait face à l'espace interne (S) du conduit de couverture (23), dans une surface externe de la plaque de motif (13) qui est tournée vers l'extérieur dans une direction radiale du tambour d'aspiration (11),
l'orifice d'éjection d'air (13O) se situe dans la portion faisant face à l'espace interne (13SOF), et
l'orifice d'éjection d'air (130) se situe dans des positions adjacentes au trou de motif (13H) dans la direction de largeur (W).

2. Appareil (1) selon la revendication 1, dans lequel le mécanisme d'éjection d'air éjecte l'air vers l'espace interne (S) du conduit de couverture (23) par le biais de la portion de moulage (15).

3. Appareil (1) selon l'une quelconque des revendications 1 à 2, dans lequel l'espace interne (S) du conduit de couverture (23) est créé pour avoir une pression négative par une aspiration du tambour d'aspiration (11), et
le mécanisme d'éjection d'air inclut en outre un orifice d'admission d'air (13I) qui est raccordé à l'orifice d'éjection d'air (13O) et est libéré dans une atmosphère.

4. Appareil selon la revendication 3, dans lequel une pluralité des orifices d'éjection d'air (13O) sont prévus, et
l'un des orifices d'éjection d'air (13O) n'est pas raccordé à un autre orifice d'éjection d'air (13O), dont la période de démarrage et une période de fin de face à face avec l'espace interne (S) du conduit de couverture (23), ne coïncident pas avec une période de démarrage et une période de fin de face à face avec l'espace interne (S) du conduit de couverture (23) du un des orifices d'éjection d'air (13O), respectivement.

5. Appareil (1) selon l'une quelconque des revendications 1 à 2, dans lequel le mécanisme d'éjection d'air inclut un mécanisme d'alimentation en air (41) qui apporte de l'air comprimé depuis un orifice d'admission d'air (13I) qui est raccordé à l'orifice d'éjection d'air (13O) vers l'orifice d'éjection d'air (13O).

6. Appareil (1) selon la revendication 5, dans lequel une pluralité des orifices d'admission d'air (13I) sont prévus, et
le mécanisme d'alimentation en air (41) apporte l'air comprimé uniquement à un orifice d'admission d'air (13I) qui est raccordé à l'orifice d'éjection d'air (13O) qui fait face à l'espace interne (S) du conduit de couverture (23), parmi les orifices d'admission d'air (13I).
